# EUROPEAN PATENT SPECIFICATION

(11) **EP 1 955 658 B1**
(45) Date of publication and mention of the grant of the patent: **25.11.2009**
(21) Application number: 08002214.8
(22) Date of filing: 06.02.2008
(51) Int. Cl.: A61B 10/06, A61B 17/28

(54) **Endoscopic treatment tool**
Endoskopisches Behandlungswerkzeug
Outil de traitement endoscopique

(30) Priority: 08.02.2007 JP 2007029053
(43) Date of publication of application: 13.08.2008
(73) Proprietor: Olympus Medical Systems Corp., Tokyo 151-0072 (JP)
(72) Inventor: Suzuki, Keita, Tokyo (JP)
(74) Representative: von Hellfeld, Axel

(56) References cited:
- EP-A- 1 454 588
- DE-U1- 8 808 285
- US-A- 5 609 285
- US-A1- 2006 229 644
- US-B1- 6 443 909

## Description

### BACKGROUND OF THE INVENTION

### Field of the Invention

The present invention relates to an endoscope treatment tool.

### Description of Related Art

An endoscope treatment tool such as grasping forceps that are used with a flexible endoscope is provided with a coil sheath that consists of a wound element wire and is inserted into a body cavity via a treatment tool insertion channel of the endoscope. In order to rotate the distal end of the endoscope treatment tool around the axis in this state, normally an operating portion of the proximal side of the endoscope treatment tool is rotated. Therefore, in order to raise the rotation-following capability of the movable distal end portion, there is known one in which a multiple turn coil sheath with high rotation transmission characteristics is disposed as the coil sheath.

Here, in the case of performing the opening/closing operation of a plurality of forceps pieces by pulling an operating wire from the operating portion such as the case with forceps, a compressive force is applied in the axial direction of the coil sheath in conjunction with the opening/closing. At this time, the multiple turn coil sheath in which a plurality of element wires are wound has high rotation transmission characteristics compared to a single turn coil sheath that consists of one wound wire, but easily compresses in the axial direction. For that reason, the coil sheath becomes compressed in the axial direction, and the axial force that should be transmitted to the distal end portion declines, with the result that sufficient treatment no longer can be performed and the procedure becomes complicated. Therefore, one in which single turn coil sheaths are disposed over a plurality of layers (for example, refer to Patent Document 1) has been proposed.

Patent Document 1: Japanese Unexamined Patent Application, First Publication No. 2000-229084.

However, the endoscope treatment tool that is disclosed in Patent Document 1 has single turn coil sheaths. Therefore, although it has excellent compressive resistance in the axial direction of the coil sheath, the rotation transmission performance is still insufficient. Also, in the case of long and thin operating wires that are connected to each of the forceps pieces being plurally disposed for individually operating the plurality of forceps pieces, the operating wires interfere with each other in the coil sheath when extending and retracting the operating wires, and the rotational torque is discontinuously transmitted.

Document G 88 08 285 U1 concerns an endoscope treatment tool having first and second coil sheaths. Through the coil sheaths, an operating wire connected to a forceps is provided, whereas the forceps may be operated via the operating wire by means of an operating portion.

Document US 2006/02229644 A1 concerns a surgical instrument for invagination and fundoplication. In one embodiment, an endoscopic surgical instrument including a torsionally rigid but flexible tube, a grasping and fastening end effector and a manual actuator are disclosed. The manual actuator is coupled to the end effector by three flexible cables which extend to the flexible tube. Each of the cables is formed from an outer coil sheath and an inner pull wire.

The present invention was achieved in view of the above circumstances, and has as its object to provide an endoscope treatment tool that can increase both the rotation operability and movability of the movable distal end portion and enable a simplification of procedures.

### SUMMARY OF THE INVENTION

In order to solve the above-mentioned problems, an endoscope treatment tool having the features of claim 1 is provided.

A first aspect of the endoscope treatment tool in accordance with the present invention consists of a movable distal end portion that performs treatment on a living body; a plurality of first coil sheaths that consist of a single element wire being spirally wound, a second coil sheath that consists of a plurality of element wires being spirally wound in the same direction and is externally mounted on the first coil sheaths; a plurality of operating wires that are shaped to extend in a long and thin manner with the distal ends thereof connected to the movable distal end portion; and an operating portion that performs extending and retracting operations of the operating wires, in which the distal end of the second coil sheath being fixed to the movable distal end portion, and the base end thereof being fixed to the operating portion, and the operating wires being arranged by being passed in a movable manner through the respective first coil sheaths.

In accordance with the first aspect of the present invention, when the operating wires are made to move in the axial direction with respect to the first coil sheaths by performing an extending/retracting operation of the operating portion, and when the operating wires are made to rotate around the center axis line of the first coil sheaths in order to operate the movable distal end portion, it is possible to suitably suppress mutual interference of the operating wires.

In particular, even when a compressive force is loaded on the second coil sheath of which the distal end and the base end are fixed, it is possible to mitigate the compressive force to the second coil sheath by the first coil sheaths, which have a high compressive resistance as a result of consisting of a single element wire wound in a spiral shape. For that reason, it is possible to suitably secure the movement amount of the operating wires with respect to the coil sheaths, and possible to suitably transmit a sufficient operating force to the movable distal end portion.

When causing the movable distal end portion to rotate by rotating the operating portion about the axis, since the first coil sheaths are externally mounted on the operating wires, by minimizing twisting of the operating wires it is possible to reduce interference between the operating wires. Moreover, since the outer diameter of the second coil sheath with high rotation transmission characteristics, which resists twisting more than the first coil sheaths, is greater than the outer diameter of the first coil sheaths, it is possible to further increase the transferability of rotational torque in the second coil sheath.

Also, the endoscope treatment tool in accordance with a second aspect of the present invention is the above-described endoscope treatment tool, in which the distal ends of the first coil sheaths being connected in a freely rotatable manner to the movable distal end portion, and the base ends thereof connected in a freely rotatable manner to the operating portion.

In accordance with the second aspect of the present invention, since the first coil sheaths are disposed in a freely rotatable manner with respect to the second coil sheath, when attempting to cause the movable distal end portion to rotate by rotating the operating portion about the axis, even when the second coil sheath twists, it is possible to suppress twisting of the first coil sheaths. For that reason, it is possible to maintain high rotation transmission characteristics.

Also, the endoscope treatment tool in accordance with a third aspect of the present invention is the above-described endoscope treatment tool, in which at least one of the element wire of the first coil sheaths and the element wire of the second coil sheath having an approximately rectangular cross section.

In accordance with the third aspect of the present invention, as a result of comparing the coil sheath that is formed by the element wire with an approximately circular cross section and the coil sheath that is formed by the element wire with the approximately rectangular cross section, assuming the cross-sectional areas of the elements wires are the same, or the outer diameter or inner diameter of the coils sheaths are the same, by adjusting the width direction dimension and the height direction dimension of the element wire with the approximately rectangular cross section, it is possible to alter the diameter of the coil sheath. For example, it is possible to make the outer diameter of the coil sheath that consists of the wound element wire with the approximately rectangular cross section smaller than the outer diameter of the coil sheath that consists of the wound element wire with the approximately circular cross section, or it is possible to make the inner diameter of the coil sheath that consists of the wound element wire with the approximately rectangular cross section greater than the inner diameter of the coil sheath that consists of the wound element wire with the approximately circular cross section.

Also, the endoscope treatment tool in accordance with a fourth aspect of the present invention is the above-described endoscope treatment tool, in which the winding direction of the element wire of the first coil sheaths and the winding direction of the element wire of the second coil sheath being the same direction.

In accordance with the fourth aspect of the present invention, in the case of the winding direction of the element wires being the same as the rotation direction around the axis of the coil sheath as a whole, since the rotational force is applied to the coil sheaths along the axial direction of the element wires, it is possible to rotate the entire coil sheath in the state of little twisting.

Also, the endoscope treatment tool in accordance with a fifth aspect of the present invention is the above-described endoscope treatment tool, in which the winding direction of the element wires of the first coil sheaths and the winding direction of the element wire of the second coil sheath being mutually opposite directions.

In accordance with the fifth aspect of the present invention, whichever direction the coil sheath as a whole is made to rotate about the axis, since the rotational force is applied along the winding direction of the element wires that are wound in a direction close to this direction, it is possible to rotate the entire coil sheath in the state of little twisting.

In accordance with the present invention, it is possible to increase both the rotation operability and movability of the movable distal end portion and enable a simplification of procedures.

### BRIEF DESCRIPTION OF THE DRAWINGS

FIG 1 is a cross-sectional view that shows the side of the distal end of the endoscope forceps in accordance with a first embodiment of the present invention.
FIG 2 is a cross-sectional view that shows the central portion of the endoscope forceps in accordance with a first embodiment of the present invention.
FIG 3 is a cross-sectional view along III-III of FIG 1.
FIG 4 is a cross-sectional view along IV IV of FIG 1.
FIG 5 is a cross-sectional view that shows the side of the base end of the endoscope forceps in accordance with a first embodiment of the present invention.
FIG 6 is a partial cross-sectional view that shows the operating portion of the endoscope forceps in accordance with a first embodiment of the present invention.
FIG 7 is a cross-sectional view that shows the side of the distal end of the endoscope forceps in accordance with a second embodiment of the present invention.
FIG 8 is a cross-sectional view that shows the side of the distal end of the endoscope forceps in accordance with a third embodiment of the present invention.
FIG 9 is an outline view that shows the first coil sheaths and the second coil sheath of the endoscope forceps in accordance with a fourth embodiment of the present invention.

### DETAILED DESCRIPTION OF THE INVENTION

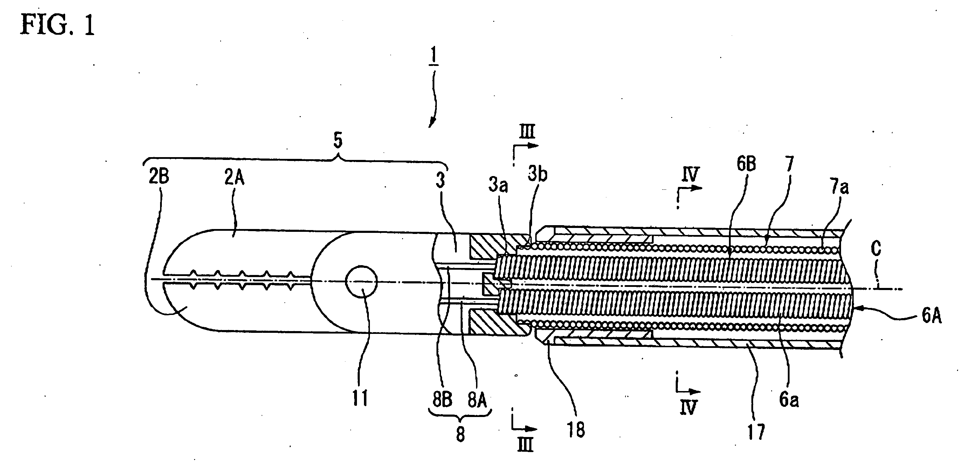

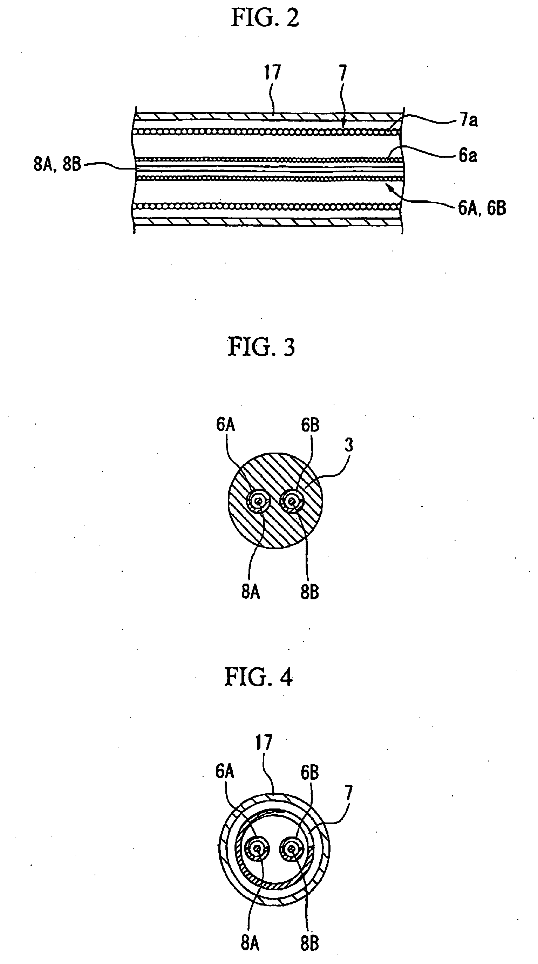

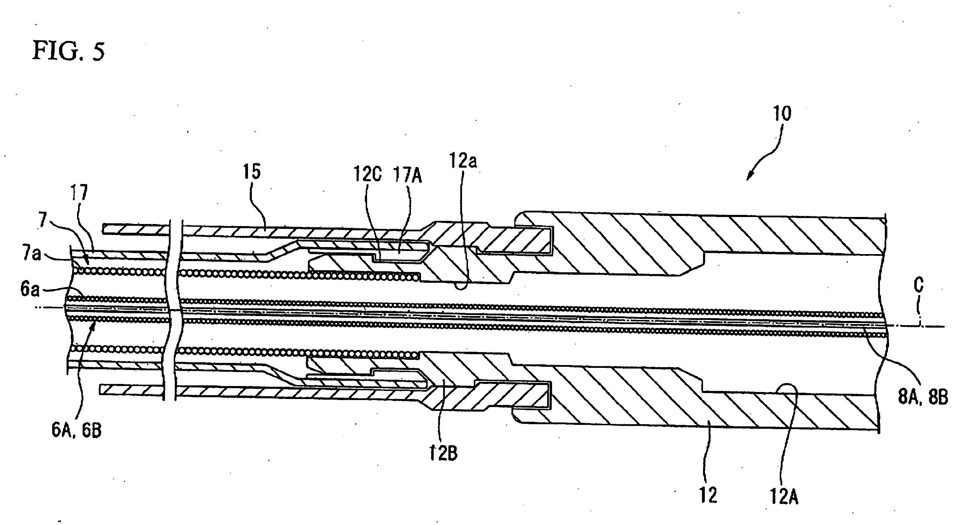

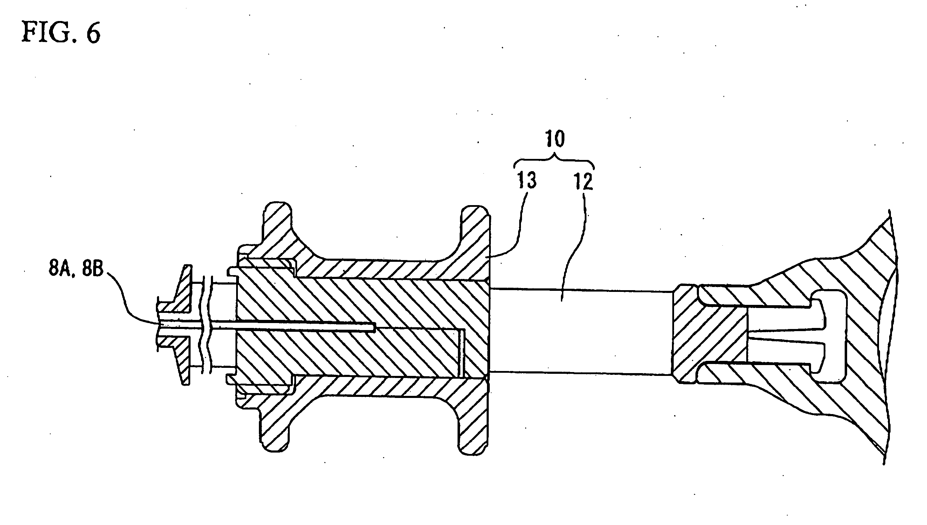

A first embodiment in accordance with the present invention shall be described with reference to FIG. 1 through FIG. 6.

An endoscope forceps (endoscope treatment tool) 1 in accordance with the present embodiment is provided with a movable distal end portion 5 that has a pair of forceps pieces 2A, 2B and a distal end cover 3 to perform treatment on a living body, two first coil sheaths 6A, 6B having flexibility that consist of a single element wire 6a being spirally wound; a second coil sheath 7 having flexibility that consists of a plurality of element wires 7a being spirally wound in the same direction and is externally mounted on the two first coil sheaths 6A, 6B; operating wires 8 having flexibility that are shaped to extend in a long and thin manner with the distal ends thereof connected to the movable distal end portion 5 and movably passed through the first coil sheaths 6A, 6B; and an operating portion 10 that performs the extending and retracting operation of the operating wires 8.

The distal end cover 3 is formed approximately cylindrical, and a supporting shaft 11 that pivotally supports mutually the pair of forceps pieces 2A, 2B is provided at the side of the distal end of the distal end cover 3 in a freely rotatable manner. Steps 3a, 3b are provided on the inside circumferential surface of the side of the base end of the distal end cover 3, the first coil sheaths 6A, 6B are connected to the step 3a, and the side of the distal end of the second coil sheath 7 is fitted to the distal end cover 3 at the step 3b.

The operating portion 10 is provided with a bar-shaped operating portion body 12 that extends in the direction of a center axis line C, and a slider 13 that is disposed to be able to freely extend and retract in the direction of the center axis line C with respect to the operating portion body 12. A slit 12A through which the operating wires 8 are passed is provided in the operating portion body 12 in the direction of the center axis line C. Also, at the side of the distal end of the operating portion body 12, a projection portion 12B is provided where the first coil sheaths 6A, 6B and the base end of the second coil sheath 7 are respectively connected to the inside circumferential surface. At the projection portion 12B, a through hole 12a that is continuous with the slit 12A is provided. The outer side of the projection portion 12B is covered by a bend preventing portion 15 for protecting the connection portion between the first coil sheaths 6A, 6B and the second coil sheath 7.

The sides of the distal end of the first coil sheaths 6A, 6B are fixed by being connected to the step 3a of the distal end cover 3, and the base ends thereof are fixed by being fitted to the inside circumferential surface of the through hole 12a of the projection portion 12B of the operating portion body 12. Since the first coil sheaths 6A, 6B are single turn coils consisting of a single element wire 6a being wound in a spiral shape, they have compressive resistance so as to resist deformation even when compressed in the direction of the center axis line C. The element wire 6a is made for example from stainless steel, and has an approximately circular cross-section.

The side of the distal end of the second coil sheath 7 is fixed by being connected at the step 3b of the distal end cover 3 in the state of a portion of the element wire 7a being shaved on the peripheral surface, and the base end thereof is fixed by being fitted to the inner circumferential surface of the through hole 12a of the projection portion 12B of the operating portion body 12. Since the second coil sheath 7 is a multiple turn coil consisting of a plurality of element wires 7a being wound in a spiral shape, rotational torque around the center axis line C becomes easily transmitted. The element wire 7a is wound in the same direction as the first coil sheaths 6, and is made for example from stainless steel, with an approximately circular cross-section.

A connection member 18 is fitted to the side of the distal end of the second coil sheath 7, with the distal end of an insulating tube 17 being externally fitted thereon. A tube-side engagement projection portion 17A is provided at the base end of the insulating tube 17, with the tube-side engagement projection portion 17A being engaged in a freely rotatable manner with an operating portion-side engagement recessed portion 12C that is provided at the projection portion 12B of the operating portion body 12.

The operating wires 8 consist of a first wire 8A that is connected in a freely rotatable manner to the base end of the forceps piece 2A and a second wire 8B that is connected in a freely rotatable manner to the base end of the forceps piece 2B. Then, both of the wires 8A, 8B are arranged by being passed through the through hole 12a of the operating portion body 12 and, by being inserted in the slit 12A, the base ends thereof are connected to the slider 13. The first wire 8A is passed through the first coil sheath 6A, and the second wire 8B is passed through the first coil sheath 6B.

Next, the action of the endoscope forceps 1 in accordance with the present embodiment shall be described.

The endoscope forceps 1 is inserted in a treatment tool insertion channel of an endoscope not illustrated that has been inserted into a body cavity in advance, and the movable distal end 5 is projected from the distal end of the endoscope to perform a predetermined treatment.

At this juncture, when the opening/closing direction of the pair of forceps pieces 2A, 2B differs from the direction in which the affected portion not illustrated should be grasped, it is necessary to adjust the endoscope forceps 1 so as to bring the directions of both into agreement. Therefore, by grasping the insulating tube 17 and rotating the operating portion 10 around the center axis line C, the opening/closing direction of the pair of forceps pieces 2A, 2B and the direction in which the affected portion should be grasped are brought into agreement.

At this time, the second coil sheath 7 has the afore-described constitution. For that reason, when the operating portion 10 is rotated by a predetermined angle, the rotational torque is transmitted to the second coil sheath 7 in the state of following the rotated angle. This rotational torque is further transmitted to the movable distal end portion 5, and so the movable distal end portion 5 rotates by the predetermined angle about the center axis line C.

After bringing the opening/closing direction of the pair of forceps pieces 2A, 2B and the direction in which the affected portion should be grasped into agreement, the slider 13 is moved to the side of the base end with respect to the operating portion body 12, and the operating wires 8 are made to move to the side of the distal end with respect to the first coil sheaths 6A, 6B and the second coil sheath 7. For that reason, the pair of forceps pieces 2A, 2B are rotated about the supporting shaft 11, leading to a state in which the pair of forceps pieces 2A, 2B are opened.

In this state, moving the slider 13 to the side of the base end with respect to the operating portion body 12 causes the first wire 8A and the second wire 8B to respectively move to the side of the base end with respect to the first coil sheaths 6A, 6B. At this time, since the respective distal ends and base ends of the first coil sheaths 6A, 6B and the second coil sheath 7 are fixed, the first coil sheaths 6A, 6B and the second coil sheath 7 are compressed in the direction of the center axis line C with the movement of the operating wires 8.

Here, since the first coil sheaths 6A, 6B have the above-described constitutions, even when the second coil sheath 7 is to be compressed by more than required, since the first coil sheaths 6A, 6B have a high compressive resistance, the second coil sheath 7 is not compressed by more than required. For that reason, use of the axial force that accompanies movement of the operating wires 8 with respect to the first coil sheaths 6A, 6B and the second coil sheath 7 for compression of the first coil sheaths 6A, 6B and the second coil sheath 7 is suppressed and transmitted to the pair of forceps pieces 2A, 2B. In this way, the pair of forceps pieces 2A, 2B is closed by respectively rotating around the supporting shaft 11 to grasp the affected portion with the required grasping force.

In accordance with this endoscope forceps 1, when the operating wires 8 are made to move in the axial direction with respect to the first coil sheaths 6, and made to rotate around the center axis line C of the first coil sheaths 6A, 6B and the second coil sheath 7 by extending or retracting the slider 13 of the operating portion 10 with respect to the operating portion body 12 in order to operate the movable distal end portion 5, it is possible to suitably suppress mutual interference of the first wire 8A and the second wire 8B within the second coil sheath 7.

Also, during this operation, even when a compressive force is loaded on the second coil sheath 7 of which the distal end and the base end are fixed, the compressive force to the second coil sheath 7 is mitigated by the first coil sheaths 6, which have a high compressive resistance as a result of consisting of a single element wire 6a wound in a spiral shape, and so sufficient operating force can be suitably transmitted to the movable distal end portion 5.

Here, the second coil sheath 7, which has high rotation transmission performance as a result of a plurality of element wires 7a being wound in a spiral shape in the same direction, is more hindered from being twisted then the first coil sheaths 6. For that reason, when causing the movable distal end portion 5 to rotate by rotating the operating portion 10 about the center axis line C, even when the first coil sheaths 6 are about to twist, it is possible to obtain a high rotation following performance. Moreover, since the outer diameter of the second coil sheath 7 is greater than the outer diameter of the first coil sheaths 6, it is possible to further increase the transferability of rotational torque in the second coil sheath 7. Accordingly, it is possible to increase both the rotation operability and movability of the movable distal end portion 5 and enable a simplification of procedures.

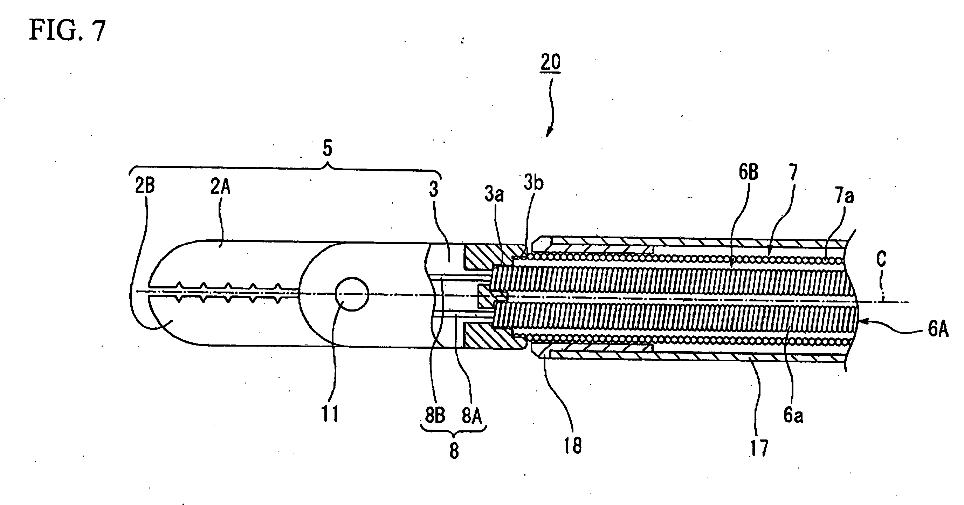

Next, a second embodiment shall be described with reference to FIG. 7.

Note that portions similar to those in the first embodiment shall be given the same reference numerals and explanations thereof shall be omitted here.

The point of difference between the second embodiment and the first embodiment is that the distal ends of the first coil sheaths 6 of an endoscope forceps 20 in accordance with the present embodiment are connected in a freely rotatable manner to the distal end cover 3 of the movable distal end portion 5, and the base ends thereof are connected in a freely rotatable manner to the projection portion 12B of the operating portion body 12 of the operating portion 10.

The action of the endoscope forceps 20 shall be described.

When the opening/closing direction of the pair of forceps pieces 2A, 2B differs from the direction in which the affected portion not illustrated should be grasped, it is necessary to adjust the endoscope forceps 1 so as to bring the directions of both into agreement. Therefore, by grasping the insulating tube 17 and rotating the operating portion 10 around the center axis line C similarly to the first embodiment, the opening/closiuag directions of the pair of forceps pieces 2A, 2B and the direction in which the affected portion should be grasped are brought into agreement.

At this time, when the operating portion 10 has been rotated by a predetermined angle with respect to the insulating tube 17, the second coil sheath 7 complies together with the movable distal end portion 5 and the operating portion 10 and rotates by the predetermined angle around the center axis line C. Meanwhile, since the first coil sheaths 6A, 6B do not co-rotate with respect to the movable distal end portion 5, the second coil sheath 7 relatively rotates with respect to the first coil sheaths 6.

In this way, after bringing the opening/closing directions of the pair of forceps pieces 2A, 2B and the direction in which the affected portion should be grasped into agreement, the opening/closing operation of the pair of forceps pieces 2A, 2B is performed by a similar operation as the first embodiment.

In accordance with this endoscope forceps 20, since the second coil sheath 7 relatively rotates with respect to the first coil sheaths 6A, 6B, it is possible to favorably suppress discontinuous rotation that occurs by repetition of storage and release of twisting that accompanies differences in the rotation angle among both.

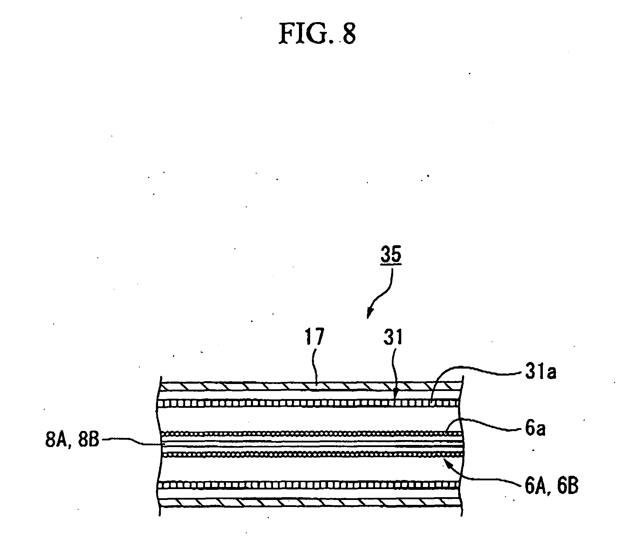

Next, a third embodiment shall be described with reference to FIG. 8.

Note that portions similar to those in other embodiments shall be given the same reference numerals and explanations thereof shall be omitted here.

The point of difference between the third embodiment and the first embodiment is that an element wire 31a of a second coil sheath 31 of an endoscope forceps 30 in accordance with the present embodiment has an approximately rectangular cross section.

In accordance with this endoscope forceps 30, as a result of comparing the second coil sheath 7, which is formed by the element wire 7a with an approximately circular cross section in accordance with the first embodiment, and the second coil sheath 31, which is formed by the element wire 31a with the approximately rectangular cross section, assuming the outer diameters of the coil sheaths are the same, the width direction dimension and the height direction dimension of the element wire 31a with the approximately rectangular cross section are adjusted. Thereby, it is possible to make the inner diameter of the second coil sheath 31 that consists of the wound element wire 31a with the approximately rectangular cross section smaller or larger than the inner diameter of the second coil sheath 7 that consists of the wound element wire with the approximately circular cross section.

Also, in the case of making the coil sheath diameters and the cross-sectional area of the element wires the same, the width direction dimension of the element wire 31 a with the approximately rectangular cross section increases more than in the case of the element wire 7a with the approximately circular cross section, and so it is possible to increase the rigidity of the coil sheath.

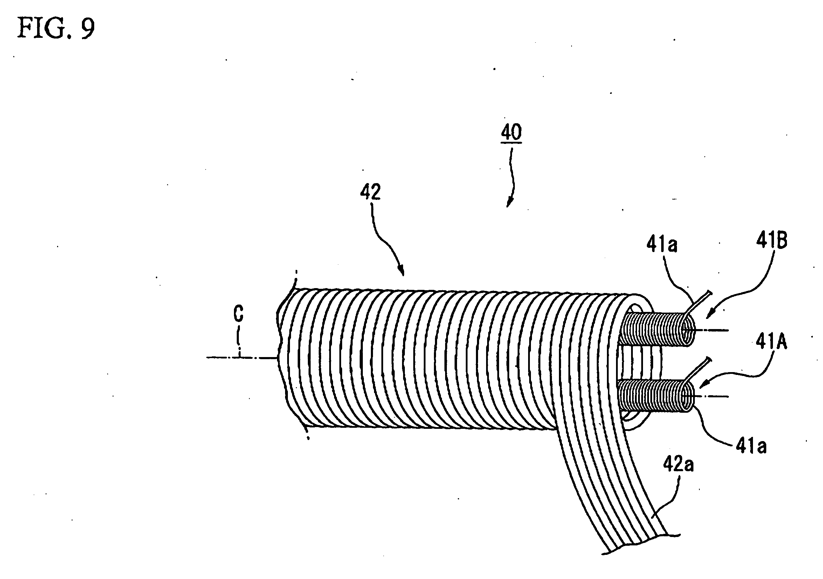

Next, a fourth embodiment shall be described with reference to FIG. 9.

Note that portions similar to those in other embodiments shall be given the same reference numerals and explanations thereof shall be omitted here.

The point of difference between the fourth embodiment and the first embodiment is that the winding direction of an element wire 41a of first coil sheaths 41A, 41B of an endoscope forceps 40 in accordance with the present embodiment and the winding direction of an element wire 42a of a second coil sheath 42 are mutually opposite directions.

That is, for example, in the case of the element wire 41 a of the first coil sheaths 41A, 41B being wound in the counterclockwise direction with respect to the center axis line C, the element wire 42a of the second coil sheath 42 is wound in the clockwise direction.

In accordance with this endoscope forceps 40, whichever direction the coil sheath as a whole is made to rotate about the axis, since the rotational force is applied along the axial direction of the element wires that are wound in a direction close to this direction, it is possible to rotate the entire coil sheath in the state of little twisting.

While preferred embodiments of the invention have been described and illustrated above, it should be understood that these are exemplary of the invention and are not to be considered as limiting. Additions, omissions, substitutions, and other modifications can be made.

For example, in the abovementioned third embodiment, the element wire 31a of the second coil sheath 31 had an approximately rectangular cross section; however, it is not limited to this. For example, it is possible to adopt an endoscope forceps in which the element wire of the first coil sheath, instead of the second coil sheath, has an approximately rectangular cross section. Also, it is possible to adopt an endoscope forceps in which either of the element wire of the first coil sheath and the element wire of the second coil sheath has an approximately rectangular cross section. In either case, it is possible to exhibit the same effect as the preferred embodiments.

## Claims

1. An endoscope treatment tool (1) comprising:
a movable distal end portion (5) that performs treatment on a living body;
a plurality of first coil sheaths (6A, 6B) that consist of a single element wire (6A) being spirally wound;
a second coil sheath (7) that consists of a plurality of element wires (7A) being spirally wound in the same direction and is externally mounted on the first coil sheaths (6A, 6B);
a plurality of operating wires (8A, 8B) that are shaped to extend in a long and thin manner with the distal ends thereof connected to the movable distal end portion (5); and
an operating portion (10) that performs extending and retracting operations of the operating wires (8A, 8B), wherein
the distal ends of the first coil sheaths (6A, 6B) are connected to the movable distal end portion (5) in a freely rotatable manner, and the base ends thereof are connected to the operating portion (10) in a freely rotatable manner,
the distal end of the second coil sheath (7) is fixed to the movable distal end portion (5), and the base end thereof is fixed to the operating portion (10), and
the operating wires (8A, 8B) are arranged by being passed in a movable manner through the respective first coil sheaths (6A, 6B).

2. The endoscope treatment tool in accordance with claim 1, wherein
at least one of the element wire of the first coil sheaths (6A, 6B) and the element wire of the second coil sheath (7A) has an approximately rectangular cross section.

3. The endoscope treatment tool in accordance with any one of claims 1 or 2, wherein
the winding direction of the element wire of the first coil sheaths (6A, 6B) and the winding direction of the element wire of the second coil sheath (7A) are the same direction.

4. The endoscope treatment tool in accordance with any one of claims 1 or 2, wherein
the winding direction of the element wires of the first coil sheaths (6A, 6B) and the winding direction of the element wire of the second coil sheath (7A) are mutually opposite directions.

## Patentansprüche

1. Endoskopbehandlungswerkzeug (1) aufweisend:
ein bewegbares Distalendteil (5), das eine Behandlung an einem lebenden Körper ausführt;
eine Mehrzahl erster Wicklungshüllen (6A, 6B), die aus einem einzelnen spiralförmig gewundenen Draht (6A) bestehen;
eine zweite Wicklungshülle (7), die aus einer Mehrzahl von in die gleiche Richtung spiralförmig gewundenen Drähten (7A) besteht, und die außen an den ersten Wicklungshüllen (6A, 6B) angebracht ist;
eine Mehrzahl von Betätigungsleitungen (8A, 8B), die so geformt sind, dass sie sich in einer langen und dünnen Weise erstrecken, wobei dessen Distalenden mit dem bewegbaren Distalendteil (5) verbunden sind; und
ein Betätigungsteil (10), das Erstreckungs- und Einziehbetätigungen der Betätigungsleitungen (8A, 8B) ausführt, wobei
die Distalenden der ersten Wicklungshüllen (6A, 6B) in einer frei drehbaren Weise mit dem bewegbaren Distalendteil (5) verbunden sind, wobei dessen Basisenden in einer frei drehbaren Weise mit dem Betätigungsteil (10) verbunden sind,
das Distalende der zweiten Wicklungshülle (7) an dem bewegbaren Distalendteil (5) befestigt ist, wobei dessen Basisende an dem Betätigungsteil (10) befestigt ist, und wobei
die Betätigungsleitungen (8A, 8B) so angeordnet sind, dass sie in einer bewegbaren Weisen durch die jeweiligen ersten Wicklungshüllen (6A, 6B) laufen.

2. Endoskopbehandlungswerkzeug gemäß Anspruch 1, bei dem
mindestens ein Draht der ersten Wicklungshüllen (6A, 6B) und der zweiten Wicklungshülle (7A) einen im Wesentlichen rechteckigen Querschnitt aufweist.

3. Endoskopbehandlungswerkzeug gemäß einem der Ansprüche 1 oder 2, bei dem
die Windungsrichtung des Drahts der ersten Wicklungshüllen (6A, 6B) und die Windungsrichtung des Drahts der zweiten Wicklungshülle (7A) die gleiche Richtung aufweisen.

4. Endoskopbehandlungsvorrichtung gemäß einem der Ansprüche 1 oder 2, bei dem
die Windungsrichtung der Drähte der ersten Wicklungshüllen (6A, 6B) und die Windungsrichtung des Drahts der zweiten Wicklungshülle (7A) gegenseitig entgegengesetzte Richtungen aufweisen.

## Revendications

1. Outil de traitement endoscopique (1) comprenant :
une partie d'extrémité distale mobile (5) qui exécute un traitement sur un corps vivant ;
une pluralité de premières gaines en bobine (6A, 6B) qui consistent en un élément en fil unique (6A) enroulé en spirale ;
une deuxième gaine en bobine (7) qui consiste en une pluralité d'éléments en fil (7A) enroulés en spirale dans le même sens et est montée extérieurement sur les premières gaines en bobine (6A, 6B) ;
une pluralité de fils opérationnels (8A, 8B) qui sont formés de façon à s'étendre d'une manière longue et mince avec les extrémités distales de ceux-ci connectées à la partie d'extrémité distale mobile (5) ; et
une partie opérationnelle (10) qui exécute des opérations d'extension et de rétraction des fils opérationnels (8A, 8B), dans lequel
les extrémités distales des premières gaines en bobine (6A, 6B) sont connectées à la partie d'extrémité distale mobile (5) d'une manière librement rotative, et les extrémités de base de celles-ci sont connectées à la partie opérationnelle (10) d'une manière librement rotative,
l'extrémité distale de la deuxième gaine en bobine (7) est fixée à la partie d'extrémité distale mobile (5), et l'extrémité de base de celle-ci est fixée à la partie opérationnelle (10), et
les fils opérationnels (8A, 8B) sont agencés en étant passés d'une manière mobile à travers les premières gaines en bobine (6A, 6B) respectives.

2. Outil de traitement endoscopique selon la revendication 1, dans lequel
au moins un de l'élément en fil des premières gaines en bobine (6A, 6B) et de l'élément en fil de la deuxième gaine en bobine (7A) a une section transversale approximativement rectangulaire.

3. Outil de traitement endoscopique selon l'une quelconque des revendications 1 ou 2, dans lequel
le sens d'enroulement de l'élément en fil des premières gaines en bobine (6A, 6B) et le sens d'enroulement de l'élément en fil de la deuxième gaine en bobine (7A) sont le même sens.

4. Outil de traitement endoscopique selon l'une quelconque des revendications 1 ou 2, dans lequel
le sens d'enroulement de l'élément en fil des premières gaines en bobine (6A, 6B) et le sens d'enroulement de l'élément en fil de la deuxième gaine en bobine (7A) sont des sens mutuellement opposés.
